# FASCICULE DE BREVET EUROPEEN

(11) **EP 0 728 463 B1**
(45) Date de publication et mention de la délivrance du brevet: **21.07.1999**
(21) Numéro de dépôt: 96400182.0
(22) Date de dépôt: 25.01.1996
(51) Int. Cl.: A61K 7/13

(54) **Composition pour la teinture d'oxydation des fibres kératiniques comprenant au moins deux bases d'oxydation et un coupleur indolique et procédé de teinture**
Oxidationshaarfärbezusammensetzung, enthaltend mindestens zwei Oxidationsbasen und einen Indolkuppler und Färbeverfahren
Composition for oxidation dyeing of keratinous fibers comprising at least two oxidation bases and an indole coupleur and process for dyeing

(30) Priorité: 27.02.1995 FR 9502269
(43) Date de publication de la demande: 28.08.1996
(73) Titulaire: L'OREAL, 75008 Paris (FR)
(72) Inventeur: Audousset, Marie-Pascale, F-92600 Asnières (FR); De La Mettrie, Roland, F-78110 Le Vesinet (FR); Cotteret, Jean, F-78480 Verneuil-sur-Seine (FR); Grimault, Christelle, F-92300 Levallois-Perret (FR)
(74) Mandataire: Goulard, Sophie

(56) Documents cités:
- EP-A- 0 428 441
- EP-A- 0 465 340
- EP-A- 0 634 164
- DE-A- 3 031 709
- GB-A- 2 180 215

## Description

La présente invention a pour objet une composition pour la teinture d'oxydation des fibres kératiniques, en particulier des fibres kératiniques humaines telles que les cheveux, comprenant au moins deux bases d'oxydation différentes l'une de l'autre en association avec un coupleur indolique convenablement sélectionné, ainsi que le procédé de teinture mettant en oeuvre cette composition avec un agent oxydant.

Il est connu de tendre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des dérivés indoliques et en particulier le 4-hydroxyindole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevets DE 3 031 709 et FR 2 664 304, des compositions pour la teinture d'oxydation des fibres kératiniques, en milieu alcalin ou acide, contenant au moins une base d'oxydation telle que la paraphénylènediamine ou le para-aminophénol, en association avec un coupleur hétérocyclique tel que par exemple le 4-hydroxy indole. De telles compositions permettent d'atteindre des gammes variées de nuances suivant la nature des bases d'oxydation choisies, mais elles ne sont cependant pas entièrement satisfaisantes notamment du point de vue de la tenue des colorations obtenues vis à vis des diverses agressions que peuvent subir les cheveux et en particulier vis à vis de la lumière.

Or, la demanderesse vient maintenant de découvrir qu'il est possible d'obtenir de nouvelles teintures, en associant au moins deux bases d'oxydation différentes convenablement sélectionnées et un coupleur indolique convenablement sélectionné, capables d'engendrer dans des nuances variées, des colorations intenses, peu sélectives et particulièrement résistantes, notamment vis à vis de la lumière, des shampooings et de la permanente.

### Cette découverte est à la base de la présente invention.

L'invention a donc pour premier objet une composition pour la teinture d'oxydation des fibres kératiniques et en particulier des fibres kératiniques humaines telles que les cheveux contenant, dans un milieu approprié pour la teinture, l'association d'au moins deux bases d'oxydation (a₁) et (a₂) différentes l'une de l'une et d'au moins un coupleur (b), caractérisée par le fait que ladite association est choisie parmi les associations suivantes :
(a₁) : 2,6-diméthyl paraphénylènediamine,
(a₂) : para-aminophénol,
(b) : 4-hydroxy indole ;

(a₁) : 2,6-diméthyl paraphénylènediamine,
(a₂) : 4-amino 3-méthyl phénol,
(b) : 4-hydroxy indole ;

(a₁) : N,N-bis-(β-hydroxyéthyl) paraphénylènediamine,
(a₂) : para-aminophénol,
(b) : 4-hydroxy indole ;

(a₁) : N,N-bis-(β-hydroxyéthyl) paraphénylènediamine,
(a₂) : 4-amino 3-méthyl phénol,
(b) : 4-hydroxy indole ;

(a₁) : paraphénylènediamine,
(a₂) : 4-amino 3-fluoro phénol,
(b) : 4-hydroxy indole ;

(a₁) : 2,6-diméthyl paraphénylènediamine,
(a₂) : 4-amino 3-fluoro phénol,
(b) : 4-hydroxy indole ;

(a₁) : 2-β-hydroxyéthyl paraphénylènediamine,
(a₂) : 2,6-diméthyl paraphénylènediamine,
(b) : 4-hydroxy indole ;

(a₁) : 2-β-hydroxyéthyl paraphénylènediamine,
(a₂) : 4-amino 3-fluoro phénol,
(b) : 4-hydroxy indole ;

(a₁) : N,N-bis-(β-hydroxyéthyl) paraphénylènediamine,
(a₂) : 4-amino 3-fluoro phénol,
(b) : 4-hydroxy indole ;

(a₁) : paratoluylènediamine,
(a₂) : 4-amino 3-fluoro phénol,
(b) : 4-hydroxy indole ;

(a₁) : 4-amino 3-fluoro phénol,
(a₂) : 4-amino 3-méthyl phénol,
(b) : 4-hydroxy indole ;

(a₁) : paraphénylènediamine,
(a₂) : 2-amino phénol,
(b) : 4-hydroxy indole ;

(a₁) : para-aminophénol,
(a₂) : 2-amino phénol,
(b) : 4-hydroxy indole ;

(a₁) : paratoluylènediamine,
(a₂) : 2-amino phénol,
(b) : 4-hydroxy indole ;

(a₁) : 2,6-diméthyl paraphénylènediamine
(a₂) : 2-amino phénol,
(b) : 4-hydroxy indole ;

(a₁) : 2-β-hydroxyéthyl paraphénylènediamine,
(a₂) : 2-amino phénol,
(b) : 4-hydroxy indole ;

(a₁) : N,N-bis-(β-hydroxyéthyl) paraphénylènediamine,
(a₂) : 2-amino phénol,
(b) : 4-hydroxy indole ;

(a₁) : 4-amino 3-méthyl phénol,
(a₂) : 2-amino phénol,
(b) : 4-hydroxy indole ;

(a₁) : 4-amino 3-fluoro phénol,
(a₂) : 2-amino phénol,
(b) : 4-hydroxy indole ;

(a₁) : paraphénylènediamine,
(a₂) :para-aminophénol,
(b) : 4-hydroxy 1-méthyl indole ;

(a₁) : paraphénylènediamine
(a₂) : 4-amino 3-méthyl phénol
(b) : 4-hydroxy 1-méthyl indole ;

(a₁) : 2,6-diméthyl paraphénylènediamine,
(a₂) : para-aminophénol,
(b) : 4-hydroxy 1-méthyl indole ;

(a₁) : 2,6-diméthyl paraphénylènediamine,
(a₂) : 4-amino 3-méthyl phénol,
(b) : 4-hydroxy 1-méthyl indole ;

(a₁) : 2-méthyl paraphénylènediamine,
(a₂) : para-aminophénol,
(b) : 4-hydroxy 1-méthyl indole ;

(a₁) : 2-méthyl paraphénylènediamine,
(a₂) : 4-amino 3-méthyl phénol,
(b) : 4-hydroxy 1-méthyl indole ;

(a₁) : paraphénylènediamine,
(a₂) : 4-amino 3-fluoro phénol,
(b) : 4-hydroxy 1-méthyl indole ;

(a₁) : paraphénylènediamine,
(a₂) : N,N'-bis-(β-hydroxyéthyl) N,N'-bis-(4'-aminophényl) 1,3-diamino 2-propanol,
(b) : 4-hydroxyindole ;

(a₁) : paraphénylènediamine,
(a₂) : N,N-bis-(hydroxyéthyl) paraphénylènediamine,
(b) : 4-hydroxyindole ;

(a₁) : 2,6-diméthyl paraphénylènediamine,
(a₂) : N,N-bis-(hydroxyéthyl) paraphénylènediamine,
(b) : 4-hydroxyindole ;

(a₁) : paraphénylènediamine,
(a₂) : 4-amino 3-hydroxyméthyl phénol,
(b) : 4-hydroxyindole ;

(a₁) : 2,6-diméthyl paraphénylènediamine,
(a₂) : 4-amino 3-hydroxyméthyl phénol,
(b) : 4-hydroxyindole ;

(a₁) : paraphénylènediamine,
(a₂) : N,N'-bis-(β-hydroxyéthyl) N,N'-bis-(4'-aminophényl) 1,3-diamino 2-propanol,
(b) : 4-hydroxy 1-méthyl indole ;

(a₁) : paraphénylènediamine,
(a₂) : N,N-bis-(hydroxyéthyl) paraphénylènediamine,
(b) : 4-hydroxy 1-méthyl indole ;

(a₁) : 2,6-diméthyl paraphénylènediamine,
(a₂) : N,N-bis-(hydroxyéthyl) paraphénylènediamine,
(b) : 4-hydroxy 1-méthyl indole;

(a₁) : paraphénylènediamine,
(a₂) : 4-amino 3-hydroxyméthyl phénol,
(b) : 4-hydroxy 1-méthyl indole ;

(a₁) : 2,6-diméthyl paraphénylènediamine,
(a₂) : 4-amino 3-hydroxyméthyl phénol,
(b) : 4-hydroxy 1-méthyl indole ;

et leurs sels d'addition avec un acide.

La composition de teinture d'oxydation conforme à l'invention permet d'obtenir des colorations aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables, notamment en ce qui concerne la résistance des colorations obtenues vis à vis de la lumière, des shampooings et des opérations de permanente.

L'invention a également pour objet un procédé de teinture d'oxydation des fibres kératiniques mettant en oeuvre cette composition.

L'ensemble des bases d'oxydation conformes à l'invention représente de préférence de 0,0005 à 12 % en poids environ du poids total de la composition tinctoriale, et encore plus préférentiellement de 0,005 à 6 % en poids environ de ce poids.

Le ou les coupleurs conformes à l'invention représentent de préférence de 0,0001 à 5 % en poids environ du poids total de la composition tinctoriale et encore plus préférentiellement de 0,005 à 3 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 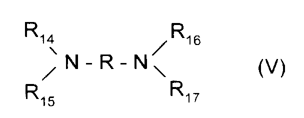 dans laquelle R est un reste propylène éventuellement substitué par un groupement hydroxyle ou un radical alkyle en C₁-C₄ ; R₁₄, R₁₅, R₁₆ et R₁₇, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, d'autres bases d'oxydation et/ou d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à l'association ternaire conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince de nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11 environ. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 3 DE TEINTURE EN MILIEU ALCALIN

On a réalisé les compositions tinctoriales conformes à l'invention suivantes (teneurs en grammes) :

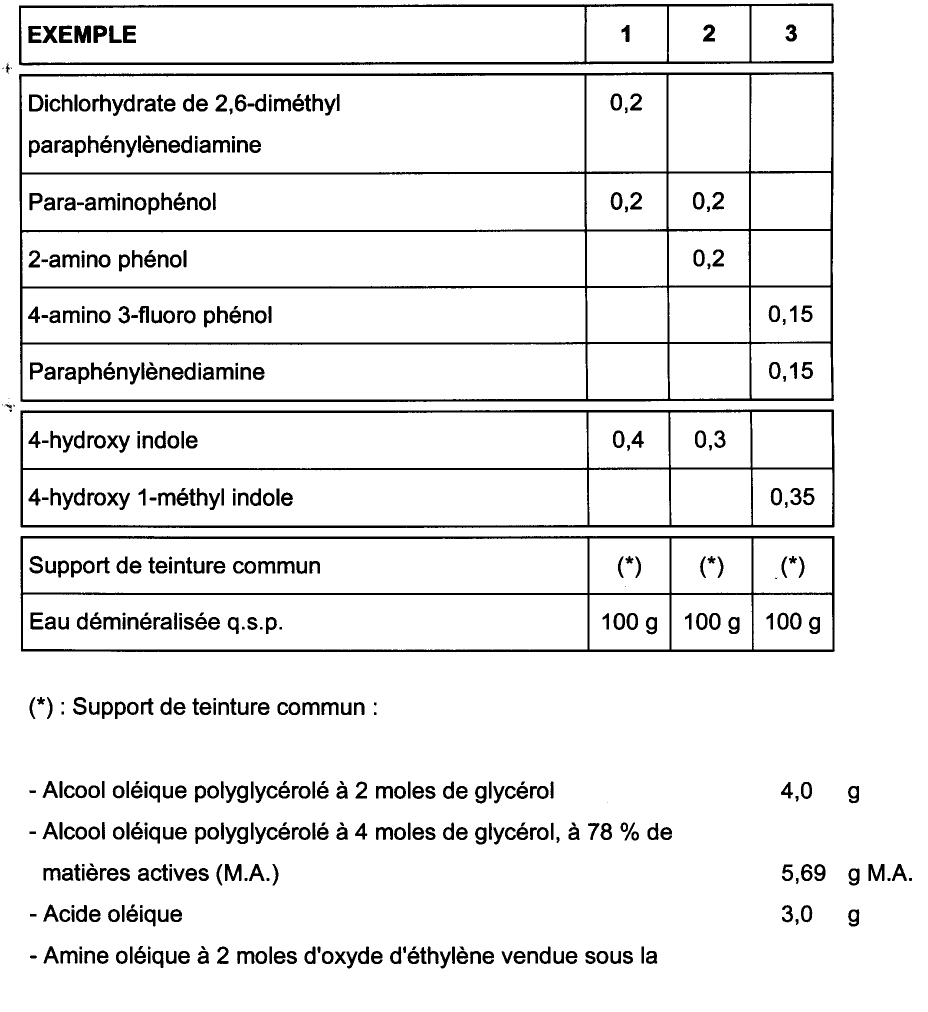

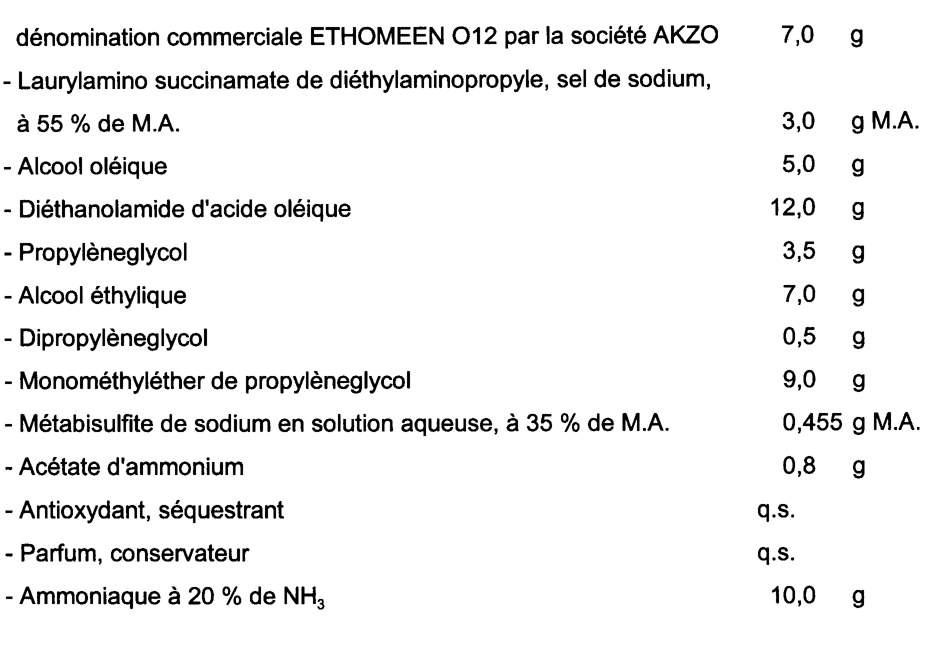

Au moment de l'emploi, on a mélangé chaque composition tinctoriale avec une quantité égale en poids d'une solution d'eau oxygénée à 20 volumes (6 % en poids).

Chaque composition résultante présentait un pH d'environ 10,2 et a été appliquée pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous

| **EXEMPLE** | **NUANCE SUR CHEVEUX NATURELS** | **NUANCE SUR CHEVEUX PERMANENTES** |
|---|---|---|
| **1** | irisé violacé légèrement cuivré | irisé légèrement violacé |
| **2** | irisé légèrement cuivré | irisé violacé légèrement cuivré |
| **3** | cendré violine | violine |

### EXEMPLE 4 DE TEINTURE EN MILIEU ACIDE

On a réalisé la composition tinctoriale conforme à l'invention suivante (teneurs en grammes) :

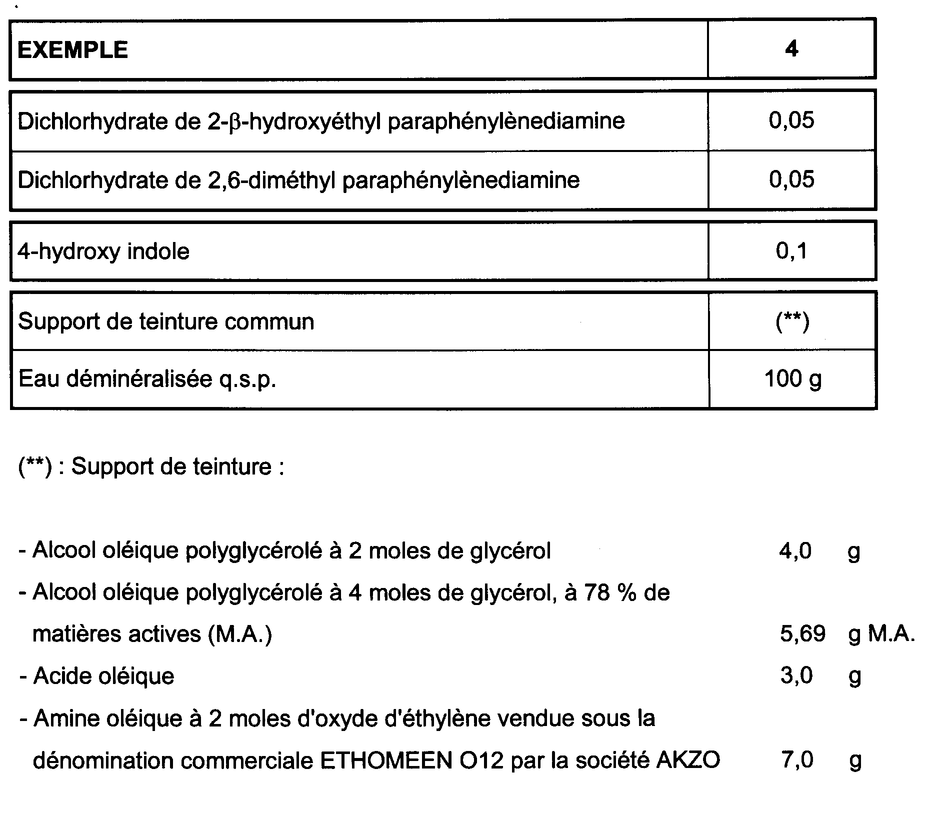

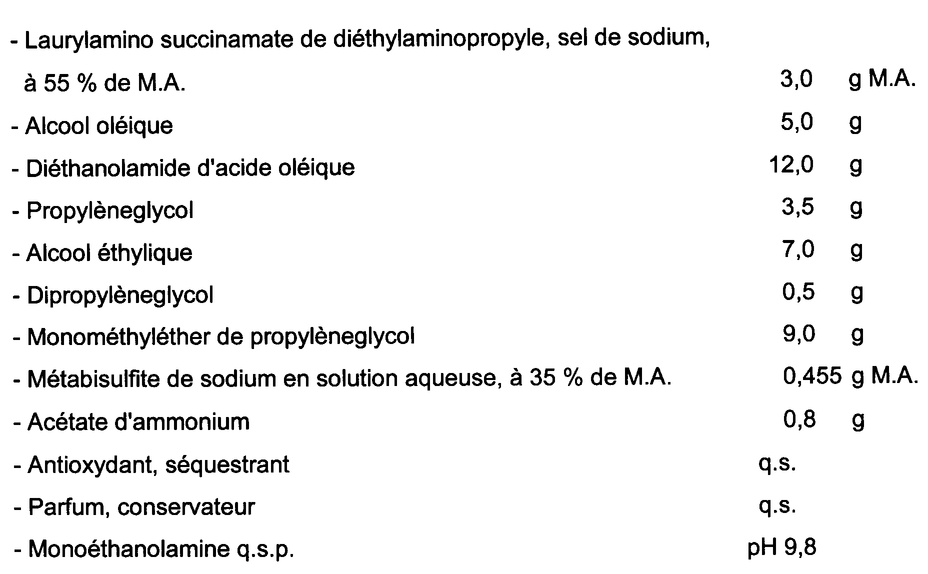

Au moment de l'emploi, on a mélangé la composition tinctoriale avec une quantité égale en poids d'une solution d'eau oxygénée à 20 volumes (6 % en poids), et dont le pH avait été ajusté entre 1 et 1,5 par 2,5 g d'acide orthophosphorique pour 100 g d'eau oxygénée.

La composition résultante présentait un pH de 6,5 et a été appliquée pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** | **NUANCE SUR CHEVEUX PERMANENTES** |
|---|---|---|
| **4** | gris naturel cendré | gris naturel légèrement bleuté |

## Revendications

1. Composition pour la teinture d'oxydation des fibres kératiniques et en particulier des fibres kératiniques humaines telles que les cheveux contenant, dans un milieu approprié pour la teinture, l'association d'au moins deux bases d'oxydation (a₁) et (a₂) différentes l'une de l'une et d'au moins un coupleur (b), caractérisée par le fait que ladite association est choisie parmi les associations suivantes :
(a₁) : 2,6-diméthyl paraphénylènediamine,
(a₂) : para-aminophénol,
(b) : 4-hydroxy indole;
(a₁) : 2,6-diméthyl paraphénylènediamine,
(a₂) : 4-amino 3-méthyl phénol,
(b) : 4-hydroxy indole;
(a₁) : N,N-bis-(β-hydroxyéthyl) paraphénylènediamine,
(a₂) : para-aminophénol,
(b) : 4-hydroxy indole ;
(a₁) : N,N-bis-(β-hydroxyéthyl) paraphénylènediamine,
(a₂) : 4-amino 3-méthyl phénol,
(b) : 4-hydroxy indole;
(a₁) : paraphénylènediamine,
(a₂) : 4-amino 3-fluoro phénol,
(b) : 4-hydroxy indole;
(a₁) : 2,6-diméthyl paraphénylènediamine,
(a₂) : 4-amino 3-fluoro phénol,
(b) : 4-hydroxy indole ;
(a₁) : 2-β-hydroxyéthyl paraphénylènediamine,
(a₂) : 2,6-diméthyl paraphénylènediamine,
(b) : 4-hydroxy indole ;
(a₁) : 2-β-hydroxyéthyl paraphénylènediamine,
(a₂) : 4-amino 3-fluoro phénol,
(b) : 4-hydroxy indole ;
(a₁) : N,N-bis-(β-hydroxyéthyl) paraphénylènediamine,
(a₂) : 4-amino 3-fluoro phénol,
(b) : 4-hydroxy indole ;
(a₁) : paratoluylènediamine,
(a₂) : 4-amino 3-fluoro phénol,
(b) : 4-hydroxy indole ;
(a₁) : 4-amino 3-fluoro phénol,
(a₂) : 4-amino 3-méthyl phénol,
(b) : 4-hydroxy indole ;
(a₁) : paraphénylènediamine,
(a₂) : 2-amino phénol,
(b) : 4-hydroxy indole ;
(a₁) : para-aminophénol,
(a₂) : 2-amino phénol,
(b) : 4-hydroxy indole ;
(a₁) : paratoluylènediamine,
(a₂) : 2-amino phénol,
(b) : 4-hydroxy indole ;
(a₁) : 2,6-diméthyl paraphénylènediamine
(a₂) : 2-amino phénol,
(b) : 4-hydroxy indole ;
(a₁) : 2-β-hydroxyéthyl paraphénylènediamine,
(a₂) : 2-amino phénol,
(b) : 4-hydroxy indole ;
(a₁) : N,N-bis-(β-hydroxyéthyl) paraphénylènediamine,
(a₂) : 2-amino phénol,
(b) : 4-hydroxy indole ;
(a₁) : 4-amino 3-méthyl phénol,
(a₂) : 2-amino phénol,
(b) : 4-hydroxy indole ;
(a₁) : 4-amino 3-fluoro phénol,
(a₂) : 2-amino phénol,
(b) : 4-hydroxy indole ;
(a₁) : paraphénylènediamine,
(a₂) :para-aminophénol,
(b) : 4-hydroxy 1-méthyl indole ;
(a₁) : paraphénylènediamine
(a₂) : 4-amino 3-méthyl phénol
(b) : 4-hydroxy 1-méthyl indole ;
(a₁) : 2,6-diméthyl paraphénylènediamine,
(a₂) : para-aminophénol,
(b) : 4-hydroxy 1-méthyl indole ;
(a₁) : 2,6-diméthyl paraphénylènediamine,
(a₂) : 4-amino 3-méthyl phénol,
(b) : 4-hydroxy 1-méthyl indole ;
(a₁) : 2-méthyl paraphénylènediamine,
(a₂) : para-aminophénol,
(b) : 4-hydroxy 1-méthyl indole ;
(a₁) : 2-méthyl paraphénylènediamine,
(a₂) : 4-amino 3-méthyl phénol,
(b) : 4-hydroxy 1-méthyl indole ;
(a₁) : paraphénylènediamine,
(a₂) : 4-amino 3-fluoro phénol,
(b) : 4-hydroxy 1 -méthyl indole ;
(a₁) : paraphénylènediamine,
(a₂) : N,N'-bis-(β-hydroxyéthyl) N,N'-bis-(4'-aminophényl) 1,3-diamino 2-propanol,
(b) : 4-hydroxyindole ;
(a₁) : paraphénylènediamine,
(a₂) : N,N-bis-(hydroxyéthyl) paraphénylènediamine,
(b) : 4-hydroxyindole ;
(a₁) : 2,6-diméthyl paraphénylènediamine,
(a₂) : N,N-bis-(hydroxyéthyl) paraphénylènediamine,
(b) : 4-hydroxyindole ;
(a₁) : paraphénylènediamine,
(a₂) : 4-amino 3-hydroxyméthyl phénol,
(b) : 4-hydroxyindole ;
(a₁) : 2,6-diméthyl paraphénylènediamine,
(a₂) : 4-amino 3-hydroxyméthyl phénol,
(b) : 4-hydroxyindole ;
(a₁) : paraphénylènediamine,
(a₂) : N,N'-bis-(β-hydroxyèthyl) N,N'-bis-(4'-aminophényl) 1,3-diamino 2-propanol,
(b) : 4-hydroxy 1-méthyl indole ;
(a₁) : paraphénylènediamine,
(a₂) : N,N-bis-(hydroxyéthyl) paraphénylènediamine,
(b) : 4-hydroxy 1-méthyl indole ;
(a₁) : 2,6-diméthyl paraphénylènediamine,
(a₂) : N,N-bis-(hydroxyéthyl) paraphénylènediamine,
(b) : 4-hydroxy 1-méthyl indole ;
(a₁) : paraphénylènediamine,
(a₂) : 4-amino 3-hydroxyméthyl phénol,
(b) : 4-hydroxy 1-méthyl indole ;
(a₁) : 2,6-diméthyl paraphénylènediamine,
(a₂) : 4-amino 3-hydroxyméthyl phénol,
(b) : 4-hydroxy 1-méthyl indole ;
et leurs sels d'addition avec un acide.

2. Composition selon la revendication 1, caractérisée par le fait que les sels d'addition avec un acide sont choisis parmi les chlorhydrates, les bromhydrates, les sulfates et les tartrates.

3. Composition selon la revendication 1 ou 2, caractérisée par le fait que l'ensemble des bases d'oxydation représente de 0,0005 à 12 % en poids du poids total de la composition tinctoriale.

4. Composition selon la revendication 3, caractérisée par le fait que l'ensemble des bases d'oxydation représente de 0,005 à 6 % en poids du poids total de la composition tinctoriale.

5. Composition selon l'une quelconque des revendications précédentes, caractérisée par le fait que le ou les coupleurs représentent de 0,0001 à 5 % en poids du poids total de la composition tinctoriale.

6. Composition selon la revendication 5, caractérisée par le fait que le ou les coupleurs représentent de 0,005 à 3 % en poids du poids total de la composition tinctoriale.

7. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

8. Composition selon l'une quelconque des revendications précédentes, caractérisée par le fait qu'elle présente un pH compris entre 3 et 12.

9. Procédé de teinture des fibres kératiniques et en particulier des fibres kératiniques humaines telles que les cheveux, caractérisé par le fait que l'on applique sur ces fibres une composition tinctoriale telle que définie à l'une quelconque des revendications 1 à 8 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

10. Procédé selon la revendication 9, caractérisé par le fait que l'agent oxydant est choisi parmi le peroxyde d'hydrogène, le peroxyde d'urée, les bromates de métaux alcalins, les persels tels que les perborates et persulfates.

11. Dispositif à plusieurs compartiments, ou "kit" de teinture à plusieurs compartiments, dont un premier compartiment renferme une composition tinctoriale telle que définie à l'une quelconque des revendications 1 à 8 et un second compartiment renferme une composition oxydante.

## Claims

1. Composition for the oxidation dyeing of keratin fibres, and in particular human keratin fibres such as the hair containing, in a medium which is suitable for dyeing, a combination of at least two oxidation bases (a₁) and (a₂) which are different from each other and of at least one coupler (b), characterized in that the said combination is chosen from the following combinations;
(a₁) : 2,6-dimethyl-para-phenylenediamine,
(a₂) : para-aminophenol,
(b) : 4-hydroxyindole;
(a₁) : 2,6-dimethyl-para-phenylenediamine,
(a₂) : 4-amino-3-methylphenol,
(b) : 4-hydroxyindole;
(a₁) : N,N-bis(β-hydxoxyethyl)-para-phenylenediamine,
(a₂) : para-aminophenol,
(b) : 4-hydroxyindole;
(a₁) : N,N-bis(β-hydroxyethyl)-para-phenylenediamine,
(a₂) : 4-amino-3-methylphenol,
(b) : 4-hydroxyindole;
(a₁) : para-phenylenediamine,
(a₂) : 4-amino-3-fluorophenol,
(b) : 4-hydroxyindole;
(a₁) : 2,6-dimethyl-para-phenylenediamine,
(a₂) : 4-amino-3-fluorophenol,
(b) : 4-hydroxyindole;
(a₁) : 2-β-hydroryethyl-para-phenylenadiamine,
(a₂) : 2,6-dimethyl-para-phenylenediamine,
(b) : 4-hydroxyindole;
(a₁) : 2-β-hydroxyethyl-para-phenylenediamine,
(a₂) : 4-amino-3-fluorophenol,
(b) : 4-hydroxyindole;
(a₁) : N,N-bis(β-hydroxyethyl)-para-phenylenediamine,
(a₂) : 4-amino-3-fluorophenol,
(b) : 4-hydroxyindole;
(a₁) : para-toluylenediamine,
(a₂) : 4-amino-3-fluorophenol,
(b) : 4-hydroxyindole;
(a₁) : 4-amino-3-fluorophenol,
(a₂) : 4-amino-3-methylphenol,
(b) : 4-hydroxyindole;
(a₁) : para-phenylenediamine,
(a₂) : 2-aminophenol,
(b) : 4-hydroxyindole;
(a₁) : para-aminophenol,
(a₂) : 2-aminophenol,
(b) : 4-hydroxyindole;
(a₁) : para-toluylenadiamine,
(a₂) : 2-aminophenol,
(b) : 4-hydroxyindole;
(a₁) : 2,6-dimethyl-para-phenylenediamine,
(a₂) : 2-aminophenol,
(b) : 4-hydroxyindole;
(a₁) : 2-β-hydroxyethyl-para-phenylenediamine,
(a₂) : 2-aminophenol,
(b) : 4-hydroxyindole;
(a₁) : N,N-bis(β-hydroxyethyl)-para-phenylenediamine,
(a₂) : 2-aminophenol,
(b) : 4-hydroxyindole;
(a₁) : 4-amino-3-methylphenol,
(a₂) : 2-aminophenol,
(b) : 4-hyroxyindole;
(a₁) : 4-amino-3-fluorophenol,
(a₂) : 2-aminophenol,
(b) : 4-hydroxyindole;
(a₁) : para-phenylenediamine,
(a₂) : para-aminophenol,
(b) : 4-hydroxy-1-methylindole;
(a₁) : para-phenylenediamine,
(a₂) : 4-amino-3-methylphenol,
(b) : 4-hydroxy-1-methylindole;
(a₁) : 2,6-dimethyl-para-phenylenediamine,
(a₂) : para-aminophenol,
(b) : 4-hydroxy-1-methylindole;
(a₁) : 2,6-dimethyl-para-phenylenediamine,
(a₂) : 4-amino-3-methylphenol,
(b) : 4-hydroxy-1-methylindole;
(a₁) : 2-methyl-para-phenylenediamine,
(a₂) : para-aminophenol,
(b) : 4-hydroxy-1-methylindole;
(a₁) : 2-methyl-para-phenylenediamine,
(a₂) : 4-amino-3-methylphenol,
(b) : 4-hydroxy-1-methylindole;
(a₁) : para-phenylenediamine,
(a₂) : 4-amino-3-fluorophenol,
(b) : 4-hydroxy-1-methylindole;
(a₁) : para-phenylenediamine,
(a₂) : N,N'-bis(β-hydroxyethyl)-N,N'-bis(4'-aminophenyl)-1,3-diamino-2-propanol,
(b) : 4-hydroxyindole;
(a₁) : para-phenylenediamine,
(a₂) : N,N-bis(hydroxyethyl)-para-phenylenediamine
(b) : 4-hydroxyindole;
(a₁) : 2,6-dimethyl-para-phenylenediamine,
(a₂) : N,N-bis(hydroxyethyl)-para-phenylenediamine
(b) : 4-hydroxyindole;
(a₁) : para-phenylenediamine,
(a₂) : 4-amino-3-hydroxymethylphenol,
(b) : 4-hydroxyindole;
(a₁) : 2,6-dimethyl-para-phenylenediamine,
(a₂) : 4-amino-3-hydroxymethylphenol,
(b) : 4-hydroxyindole;
(a₁) : para-phenylenediamine,
(a₂) : N,N'-bis(β-hydxoyethyl)-N,N'-bis(4'-aminophenyl)-1,3-diamino-2-prapanol,
(b) : 4-hydroxy-1-methylindole;
(a₁) : para-phenylenediamine,
(a₂) : N,N-bis(hydroxyethyl)-para-phenylenediamine,
(b) : 4-hydroxy-1-methylindole;
(a₁) : 2,6-dimethyl-para-phenylenediamine,
(a₂) : N,N-bis(hydroxyethyl)-para-phenylenediamine,
(b) : 4-hydroxy-1-methylindole;
(a₁) : para-phenylenediamine,
(a₂) : 4-amino-3-hydroxymethylphenol,
(b) : 4-hydroxy-1-methylindole;
(a₁) : 2,6-dimethyl-para-phenylenediamine,
(a₂) : 4-amino-3-hydroxmethylphenol,
(b) : 4-hydroxy-1-methylindole;
and the addition salts thereof with an acid.

2. Composition according to Claim 1, characterized in that the addition salts with an acid are chosen from the hydrochlorides, hydrobromides, sulphates and tartrates.

3. Composition according to Claim 1 or 2, characterized in that the oxidation bases together represent tram 0.0005 to 12 % by weight relative to the total weight of the dye composition.

4. Composition according to Claim 3, characterized in that the oxidation bases together represent from 0.005 to 6 % by weight relative to the total weight of the dye composition.

5. Composition according to any one of the preceding claims, characterized in that the coupler or couplers represent from 0.0001 to 5 % by weight relative to the total weight of the dye composition.

6. Composition according to Claim 5, characterized in that the coupler or couplers rePresent from 0.005 to 3 % by weight relative to the total weight of the dye composition.

7. Composition according to any one of the preceding claims, characterized in that the medium which is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

8. Composition according to any one of the preceding claims, characterized in that it has a pH of between 3 and 12.

9. Process for dyeing keratin fibres, and in particular human keratin fibres such as the hair, characterized in that a dye composition as defined in any one of claims 1 to 8 is applied to these fibres and in that the colour is developed at acidic, neutral or alkaline pH using an oxidizing agent which is added only at the time of use to the dye composition or which is present in an oxidizing composition that is applied simultaneously or sequentially in a separate manner.

10. Process according to Claim 9, characterized in that the oxidizing agent is chosen from hydrogen peroxide, urea peroxide, alkali metal bromates and persalts such as perborates and persulphates.

11. Multi-compartment device, or multi-compartment dyeing "kit", a first compartment of which contains a dye composition as defined in any one of Claims 1 to 8 and a second compartment of which contains an oxidizing composition.

## Patentansprüche

1. Zusammensetzung zur oxidativen Färbung von Keratinfasern und insbesondere von menschlichen Keratinfasern, wie z.B. der Haare, die in einem für die Färbung geeigneten Medium die Kombination aus mindestens zwei verschiedenen Oxidationsbasen (a₁) und (a₂) und mindestens einem Kuppler (b) enthält, dadurch gekennzeichnet ist, daß die Kombination unter den folgenden Kombinationen ausgewählt ist:
(a₁) : 2,6-Dimethyl-p-phenylendiamin,
(a₂) : p-Aminophenol,
(b) : 4-Hydroxyindol;
(a₁) : 2,6-Dimethyl-p-phenylendiamin,
(a₂) : 4-Amino-3-methylphenol,
(b) : 4-Hydroxyindol;
(a₁) : N,N-Bis(β-hydroxyethyl)-p-phenylendiamin,
(a₂) : p-Aminophenol,
(b) : 4-Hydroxyindol;
(a₁) : N,N-Bis(β-hydroxyethyl)-p-phenylendiamin,
(a₂) : 4-Amino-3-methylphenol,
(b) : 4-Hydroxyindol;
(a₁) : p-Phenylendiamin,
(a₂) : 4-Amino-3-fluorphenol,
(b) : 4-Hydroxyindol;
(a₁) : 2,6-Dimethyl-p-phenylendiamin,
(a₂) : 4-Amino-3-fluorphenol,
(b) : 4-Hydroxyindol;
(a₁) : 2-β-Hydroxyethyl-p-phenylendiamin,
(a₂) : 2,6-Dimethyl-p-phenylendiamin,
(b) : 4-Hydroxyindol;
(a₁) : 2-β-Hydroxyethyl-p-phenylendiamin,
(a₂) : 4-Amino-3-fluorphenol,
(b) : 4-Hydroxyindol;
(a₁) : N,N-BiS(β-hydroxyethyl)-p-phenylendiamin,
(a₂) : 4-Amino-3-fluorphenol,
(b) : 4-Hydroxyindol;
(a₁) : p-Toluylendiamin,
(a₂) : 4-Amino-3-fluorphenol,
(b) : 4-Hydroxyindol;
(a₁) : 4-Amino-3-fluorphenol,
(a₂) : 4-Amino-3-methylphenol,
(b) : 4-Hydroxyindol;
(a₁) : p-Phenylendiamin,
(a₂) : 2-Aminophenol,
(b) : 4-Hydroxyindol;
(a₁) : p-Aminophenol,
(a₂) : 2-Aminophenol,
(b) : 4-Hydroxyindol;
(a₁) : p-Toluylendiamin,
(a₂) : 2-Aminophenol,
(b) : 4-Hydroxyindol;
(a₁) : 2,6-Dimethyl-p-phenylendiamin,
(a₂) : 2-Aminophenol,
(b) : 4-Hydroxyindol;
(a₁) : 2-β-Hydroxyethyl-p-phenylendiamin,
(a₂) : 2-Aminophenol,
(b) : 4-Hydroxyindol;
(a₁) : N,N-Bis(β-hydroxyethyl)-p-phenylendiamin,
(a₂) : 2-Aminophenol,
(b) : 4-Hydroxyindol;
(a₁) : 4-Amino-3-methylphenol,
(a₂) : 2-Aminophenol,
(b) : 4-Hydroxyindol;
(a₁) : 4-Amino-3-fluorphenol,
(a₂) : 2-Aminophenol,
(b) : 4-Hydroxyindol;
(a₁) : p-Phenylendiamin,
(a₂) : p-Aminophenol,
(b) : 4-Hydroxy-1-methylindol;
(a₁) : p-Phenylendiamin,
(a₂) : 4-Amino-3-methylphenol,
(b) : 4-Hydroxy-1-methylindol;
(a₁) : 2,6-Dimethyl-p-phenylendiamin,
(a₂) : p-Aminophenol,
(b) : 4-Hydroxy-1-methylindol;
(a₁) : 2,6-Dimethyl-p-phenylendiamin,
(a₂) : 4-Amino-3-methylphenol,
(b) : 4-Hydroxy-1-methylindol;
(a₁) : 2-Methyl-p-phenylendiamin,
(a₂) : p-Aminophenol,
(b) : 4-Hydroxy-1-methylindol;
(a₁) : 2-Methyl-p-phenylendiamin,
(a₂) : 4-Amino-3-methylphenol,
(b) : 4-Hydroxy-1-methylindol;
(a₁) : p-Phenylendiamin,
(a₂) : 4-Amino-3-fluorphenol,
(b) : 4-Hydroxy-1-methylindol;
(a₁) : p-Phenylendiamin,
(a₂) : N,N'-Bis(β-hydroxyethyl)-N,N'-bis(4'-aminophenyl)-1,3-diamino-2-propanol,
(b) : 4-Hydroxyindol;
(a₁) : p-Phenylendiamin,
(a₂) : N,N-Bis(hydroxyethyl)-p-phenylendiamin,
(b) : 4-Hydroxyindol;
(a₁) : 2,6-Dimethyl-p-phenylendiamin,
(a₂) : N,N-Bis(hydroxyethyl)-p-phenylendiamin,
(b) : 4-Hydroxyindol;
(a₁) : p-Phenylendiamin,
(a₂) : 4-Amino-3-hydroxymethylphenol,
(b) : 4-Hydroxyindol;
(a₁) : 2,6-Dimethyl-p-phenylendiamin,
(a₂) : 4-Amino-3-hydroxymethylphenol,
(b) : 4-Hydroxyindol;
(a₁) : p-Phenylendiamin,
(a₂) : N,N'-BiS(β-hydroxyethyl)-N,N'-bis-(4'-aminophenyl)-1,3-diamino-2-propanol,
(b) : 4-Hydroxy-1-methylindol;
(a₁) : p-Phenylendiamin,
(a₂) : N,N-Bis(hydroxyethyl)-p-phenylendiamin,
(b) : 4-Hydroxy-1-methylindol;
(a₁) : 2,6-Dimethyl-p-phenylendiamin,
(a₂) : N,N-Bis(hydroxyethyl)-p-phenylendiamin,
(b) : 4-Hydroxy-1-methylindol;
(a₁) : p-Phenylendiamin,
(a₂) : 4-Amino-3-hydroxmethylphenol,
(b) : 4-Hydroxy-1-methylindol;
(a₁) : 2,6-Dimethyl-p-phenylendiamin,
(a₂) : 4-Amino-3-hydroxmethylphenol,
(b) : 4-Hydroxy-1-methylindol;
und ihren Additionssalzen mit einer Säure.

2. Zusammensetzung nach Anspruch 1, dadurch gekennzeichnet, daß die Additionssalze mit einer Säure unter den Hydrochloriden, den Hydrobromiden, den Sulfaten und den Tartraten ausgewählt sind.

3. Zusammensetzung nach Anspruch 1 oder 2, dadurch gekennzeichnet, daß die Oxidationsbasen zusammen 0,0005 bis 12 Gew.-% des Gesamtgewichts der Färbemittelzusammensetzung ausmachen.

4. Zusammensetzung nach Anspruch 3, dadurch gekennzeichnet, daß die Oxidationsbasen zusammen 0,005 bis 6 Gew.-% des Gesamtgewichts der Färbemittelzusammensetzung ausmachen.

5. Zusammensetzung nach einem der vorhergehenden Ansprüche, dadurchgekennzeichnet, daß der oder die Kuppler 0,0001 bis 5 Gew.-% des Gesamtgewichts der Färbemittelzusammensetzung ausmachen.

6. Zusammensetzung nach Anspruch 5, dadurch gekennzeichnet, daß der oder die Kuppler 0,005 bis 3 Gew.-% des Gesamtgewichts der Färbemittelzusammensetzung ausmachen.

7. Zusammensetzung nach einem der vorhergehenden Ansprüche, dadurch gekennzeichnet, daß das für die Färbung geeignete Medium (oder der Träger) aus Wasser oder aus einem Gemisch aus Wasser und mindestens einem organischen Lösungsmittel, das unter niederen C₁-C₄-Alkanolen, Glycerin, Glykolen und Glykolethern, aromatischen Alkoholen, analogen Verbindungen und deren Gemischen ausgewählt ist.

8. Zusammensetzung nach einem der vorhergehenden Ansprüche, dadurch gekennzeichnet, daß sie einen pH-Wert im Bereich von 3 bis 12 aufweist.

9. Verfahren zum Färben von Keratinfasern und insbesondere menschlicher Keratininfasern, wie z.B. der Haare, dadurch gekennzeichnet, daß auf die Fasern eine wie in einem der Ansprüche 1 bis 8 definierte Färbemittelzusammensetzung aufgetragen wird und daß die Farbe bei saurem, neutralem oder alkalischem pH-Wert mit Hilfe eines Oxidationsmittels entwickelt wird, das genau zum Zeitpunkt der Verwendung der Färbemittelzusammensetzung zugegeben wird oder das in einer oxidierenden Zusammensetzung enthalten ist, die gleichzeitig oder zeitlich versetzt in getrennter Weise aufgetragen wird.

10. Verfahren nach Anspruch 9 dadurch gekennzeichnet, daß das Oxidationsmittels unter Wasserstoffperoxid, Harnstoffperoxid, Alkalimetallbromaten, Persalzen, wie z.B. Perboraten und Persulfaten, ausgewählt wird.

11. Vorrichtung mit mehreren Abteilungen oder "Kit" zum Färben mit mehreren Abteilungen, von denen eine erste Abteilung eine wie in einem der Ansprüche 1 bis 8 definierte Färbemittelzusammensetzung und eine zweite Abteilung eine oxidierende Zusammensetzung enthält.
